# EUROPÄISCHE PATENTSCHRIFT

(11) **EP 0 714 890 B1**
(45) Veröffentlichungstag und Bekanntmachung des Hinweises auf die Patenterteilung: **24.04.2002**
(21) Anmeldenummer: 95115308.9
(22) Anmeldetag: 28.09.1995
(51) Int. Cl.: C07D 211/58

(54) **Verfahren zur Herstellung von 4-Amino-2,2,6,6-tetramethyl-piperidin**
Process and the preparation of 4-amino-2,2,6,6-tetra-methyl piperidine
Procédé pour la préparation de la 4-amino-2,2,6,6-tétraméthyle piperidine

(30) Priorität: 02.12.1994 DE 4442990
(43) Veröffentlichungstag der Anmeldung: 05.06.1996
(73) Patentinhaber: Degussa AG, 40474 Düsseldorf (DE)
(72) Erfinder: Frentzen, Stefan, D-46348 Raesfeld (DE); Neuber, Elmhart, Dr., D-45721 Haltern (DE); Thelen, Gerhard, Dr., D-48301 Nottuln (DE)

(56) Entgegenhaltungen:
- EP-A- 0 033 529
- DE-A- 2 412 750
- US-A- 4 923 992
- CHEMICAL ABSTRACTS, vol. 105, no. 5, 4.August 1986 Columbus, Ohio, US; abstract no. 42660g, Seite 725; Spalte 1; XP002017971 & JP-A-61 033 169 (JPN. KOKAI TOKKYO KOHO) 17.Februar 1986

## Beschreibung

Die Erfindung betrifft ein Verfahren zur Herstellung von 4-Amino-2,2,6,6-tetramethylpiperidin (im folgenden Triacetondiamin oder TAD genannt) aus 2,2,6,6-Tetramethylpiperidon (im folgenden Triacetonamin oder TAA genannt), Ammoniak und Wasserstoff unter Verwendung von Kobalt- oder Nickelkatalysatoren auf einem Trägermaterial.

Durch aminierende Hydrierung kann TAA zu TAD umgesetzt werden. Dabei wird in einem ersten Schritt unter Wasserabspaltung zunächst das entsprechende Imin aus TAA und NH₃ gebildet, welches umgesetzt wird. Die hierbei beschriebenen Reaktionen laufen im allgemeinen in einem einstufigen Prozeß ab, wobei üblicherweise Drücke von 15 - 100 bar und Temperaturen von 100 - 140 °C notwendig sind. Üblicherweise werden Kobalt- oder NickelKatalysatoren verwendet.

TAD findet als Zwischenprodukt in der Synthese der sog. HALS-Produkte (Hindered Amine Light Stabilizers) Verwendung.

Bekannte Möglichkeiten der großtechnischen Herstellung von TAD aus TAA bestehen in der Verwendung von Raney-Nickel (SU 1088304) oder Raney-Kobalt (J6 61033169) als Katalysator und arbeiten ausnahmslos mit einem zusätzlich einzubringenden Lösungsmittel, meist Methanol (Beispiele in JP 62016461 und JP 61033169); zur Umsetzung nach diesen Verfahren ist weiterhin immer ein deutlicher molarer Ammoniak-Überschuß notwendig.

Die Arbeitsweise nach dem Stand der Technik mit einem zusätzlich einzubringenden Lösungsmittel (meist Methanol oder Wasser) hat einen niedrigeren Auslastungsgrad des Reaktors und im allgemeinen aufwendigere und kostspieligere zusätzliche Schritte zur späteren Abtrennung des Lösungsmittels zur Folge. Bei organischen Lösungsmitteln (z. B. das bereits genannte Methanol) treten zusätzlich nicht unerhebliche Einsatzstoffkosten auf. Weiterhin ist das Gefahrenpotential höher als bei der Arbeitsweise ohne organische Lösungsmittel.

Die Katalysatorenmengen nach dem Stand der Technik liegen bei zum Teil bis zu 10 % (Beispiel in GB 2176473) des Einsatzstoffes (TAA) und haben entsprechende Kosten zur Folge.

Hohe Überschüsse an Ammoniak führen zu entsprechend (energie)-aufwendigen Aufarbeitungsschritten bei der Rückgewinnung des überschüssigen Ammoniaks und reduzieren ebenso das nutzbare Reaktorvolumen für den Einsatzstoff (TAA).

Es bestand daher die Aufgabe, die Herstellung von TAD und TAA zu optimieren, insbesondere
- die Raum-Zeit-Ausbeute des Reaktors durch möglichst gänzliches Weglassen des Lösungsmittels zu erhöhen
- die Katalysatormenge zu reduzieren
- den Ammoniak-Überschuß zu minimieren,
ohne daß signifikante Verschlechterungen in der Produktqualität auftreten.

Es wurde nun überraschend gefunden, daß durch die Verwendung von Kobalt- oder Nickelkatalysatoren auf einem Träger in allen genannten Punkten eine deutliche Verbesserung gegenüber dem Stand der Technik erzielt werden kann.

Gegenstand der Erfindung ist daher ein Verfahren zur Herstellung von 4-Amino-2,2,6,6-tetramethylpiperidin, wobei man 2,2,6,6-Tetra-methylpiperidon und Katalysator vorlegt und flüssiges Ammoniak und danach Wasserstoff zugibt, dadurch gekennzeichnet, daß die Reaktion in Gegenwart von Kobalt- oder Nickelkatalysatoren auf einem Trägermaterial durchgeführt wird und daß bei der Reaktion kein zusätzliches Lösungsmittel eingebracht wird.

Durch Einsparung von zusätzlichen Lösungsmitteln werden Einsatzstoffkosten und Destillationskosten reduziert und die Raum-Zeit-Ausbeute des Reaktors erhöht, da jetzt das gesamte Reaktorvolumen mit Wertprodukt gefüllt werden kann. Weiterhin wird der molare Ammoniak-Überschuß von bisher Ammoniak : TAA= 2.1 bis 2,75 : 1 (siehe Beispiel in GB 2176473 und J6 2016461) auf Werte von z. B. nur noch Ammoniak : TAA = 1.4 : 1 reduziert. Das molare Verhältnis beträgt 1 : 1 bis 2 : 1. Durch Einsparung von Ammoniak werden Einsatzstoffkosten und Aufarbeitungskosten zur Ammoniak-Rückgewinnung reduziert und ebenso die Raum-Zeit-Ausbeute noch weiter erhöht.

Die Katalysatorkonzentration beträgt 0,05 bis 0,2 %, bezogen auf eingesetztes 2,2,6,6-Tetramethylpiperidon. Der Berechnung wird dabei der Metallgehalt des Trägerkatalysators zu Grunde gelegt. Als Träger dienen beispielsweise Aluminiumoxid, Siliciumdioxid oder Zinkoxid.

Durch die Verringerung der Katalysatormenge werden die Herstellkosten reduziert. Die Temperatur bei der hydrierenden Aminierung beträgt 80 °C bis 130 °C. Der Druck bei der hydrierenden Aminierung beträgt 50 bis 150 bar.

Die nachfolgenden Beispiele sollen den Gegenstand der vorliegenden Erfindung erläutern, ohne ihn darauf einzuschränken.

### Beispiel 1:

In einen sauberen, trockenen und vorher mit Stickstoff gespülten Reaktor werden 3100 kg TAA (= 20 kmol) eingefüllt, 5.8 kg Kobaltkatalysator RCH 45/20 der Fa. Ruhrchemie (das sind 0.19 % Gesamtkatalysator bezogen auf TAA; die Kobaltkonzentration beträgt 48 % im Katalysator, das sind 5.8 kg · 48 % = 2.8 kg Kobalt, entsprechend 0.09 % Kobalt bzgl. eingesetztes TAA) werden zugefügt und homogenisiert. Anschließend wird flüssiges Ammoniak zugegeben (540 kg = 31.8 kmol), die Mischung auf 120 °C aufgeheizt und mit Wasserstoff auf 95 bar aufgedrückt. Nach 2.5 - 3 Stunden Reaktion bei 120 °C (Wärmeabfuhr ist notwendig) ist die Reaktion beendet, der Reaktorinhalt wird abgekühlt und entspannt. Man erhält ein TAD der Reinheit von 96 - 97 %. Die Aufarbeitung erfolgt über die allgemein üblichen Reinigungsschritte, zum Beispiel destillativ.

### Beispiel 2:

In einen sauberen, trockenen und vorher mit Stickstoff gespülten Reaktor werden 310 g TAA (= 2 mol) eingefüllt, 0.58 g Kobaltkatalysator RCH 45/20 der Fa. Ruhrchemie (das sind 0.19 % Gesamtkatalysator bezogen auf TAA; die Kobaltkonzentration beträgt 48 % im Katalysator, das sind 0.58 g · 48 % = 0.28 g Kobalt, entsprechend 0.09 % Kobalt bzgl. eingesetztes TAA) wird zugefügt und gerührt. Anschließend wird flüssiges Ammoniak zugegeben (46.5 g = 2.74 mol), die Mischung auf 120 °C aufgeheizt und mit Wasserstoff auf 95 bar aufgedrückt. Nach 6 h Reaktion bei 120 °C (Wärmeabfuhr ist notwendig) ist die Reaktion beendet, der Autoklav wird abgekühlt und entspannt. Man erhält ein TAD der Reinheit von 95 %. Die Aufarbeitung erfolgt über die allgemein üblichen Reinigungsschritte, zum Beispiel destillativ.

## Patentansprüche

1. Verfahren zur Herstellung von 4-Amino-2,2,6,6-tetramethylpiperidin, wobei man 2,2,6,6-Tetramethylpiperidon und Katalysator vorlegt und flüssiges Ammoniak und danach Wasserstoff zugibt,
**dadurch gekennzeichnet,**
**dass** die Reaktion in Gegenwart von 0,05 bis 0,2 % Kobalt- oder Nickelkatalysatoren, bezogen auf eingesetztes 2,2,6,6-Tetramethylpiperidon, auf einem Trägermaterial bei einem Molverhältnis Ammoniak : 2,2,6,6-Tetramethylpiperidon von 1 : 1 bis 2 : 1 bei einer Temperatur von 80 bis 130 °C und einem Druck von 50 bis 150 bar durchgeführt wird und dass bei der Reaktion kein zusätzliches Lösungsmittel eingebracht wird.

## Claims

1. A process for preparing 4-amino-2,2,6,6-tetramethylpiperidine by initially charging 2,2,6,6-tetramethylpiperidone and a catalyst and adding liquid ammonia and then hydrogen, **characterized in that** the reaction is carried out in the presence of from 0.05 to 0.2% of cobalt or nickel catalysts, based on 2,2,6,6-tetramethylpiperidone used, on a support material at an ammonia : 2,2,6,6-tetramethylpiperidone molar ratio of from 1 : 1 to 2 : 1, a temperature of from 80 to 130°C and a pressure of from 50 to 150 bar, and **in that** no additional solvent is introduced in the course of the reaction.

## Revendications

1. Procédé de préparation de la 4-amino-2,2,6,6-tétraméthylpipéridine dans lequel on introduit la 2,2,6,6-tétraméthylpipéridone et le catalyseur et on ajoute l'ammoniac liquide et ensuite l'hydrogène,
**caractérisé en ce qu'**
on effectue la réaction en présence de 0,05 à 0,2 % de catalyseurs au cobalt ou au nickel, par rapport à la 2,2,6,6-tétraméthylpipéridone mise en oeuvre, sur un matériau de support pour un rapport molaire ammoniac/2,2,6,6-tétraméthylpipéridone de 1 : 1 à 2 : 1 à une température de 80 à 130°C et sous une pression de 50 à 150 bars, et on n'introduit aucun solvant supplémentaire au cours de la réaction.
